# FASCICULE DE BREVET EUROPEEN

(11) **EP 1 453 783 B1**
(45) Date de publication et mention de la délivrance du brevet: **04.10.2006**
(21) Numéro de dépôt: 02804241.4
(22) Date de dépôt: 03.12.2002
(51) Int. Cl.: C07C 49/84, C07C 225/22, A61K 31/122, A61K 31/136, A61P 35/00

(54) **ARYL-CYCLOALCANES SUBSTITUES ET LEUR UTILISATION COMME AGENTS ANTICANCEREUX**
SUBSTITUIERTE ARYLCYCLOALKANE UND DEREN VERWENDUNG ALS ANTIKREBSMITTEL
SUBSTITUTED ARYL-CYCLOALKANES, AND USE THEREOF AS ANTICANCER AGENTS

(30) Priorité: 05.12.2001 FR 0115740
(43) Date de publication de la demande: 08.09.2004
(73) Titulaire: Aventis Pharma S.A., 92160 Antony (FR)
(72) Inventeur: MAILLIET, Patrick, F-94120 Fontenay sous Bois (FR); CAPET, Marc, F-35520 Melese (FR); TIRABOSCHI, Gilles, F-91230 Montgeron (FR); CAULFIELD, Thomas, LEBANON, NJ 08833 (US)
(86) Numéro de dépôt international: PCT/FR2002/004142
(87) Numéro de publication internationale: WO 2003/048096

(56) Documents cités:
- EP-A- 0 288 794
- DE-A- 1 935 458
- FR-A- 1 526 708
- FR-A- 1 534 252
- US-A- 3 506 670
- LETTON J C ET AL: "Synthesis of some cis- and trans-2-(substituted amino)cyclohexyl phenyl ketones" JOURNAL OF MEDICINAL CHEMISTRY, vol. 15, no. 12, décembre 1972 (1972-12), pages 1328-1331, XP002236733
- CUSSAC M ET AL: "Alcools pyridiniques cyclopropaniques" EUROPEAN JOURNAL OF MEDICINAL CHEMISTRY - CHIMICA THERAPEUTICA, vol. 12, no. 2, 1977, pages 177-181, XP002236734
- SKALETZKY L L ET AL: "The relationship between structure, stereochemistry, and diuretic activity in the 2-amino-alpha- cyclohexanemethanol series, a new class of diuretic agents. II" JOURNAL OF MEDICINAL CHEMISTRY, vol. 12, no. 6, novembre 1969 (1969-11), pages 977-988, XP002236735
- SZMUSZKOVICZ J ET AL: "Synthesis and stereochemistry of amino alcohols and derivatives in the 2-amino-alpha- phenylcyclohexanemethanol series" JOURNAL OF ORGANIC CHEMISTRY, vol. 32, no. 11, novembre 1967 (1967-11), pages 3300-3313, XP002236736
- EDWARDS M L ET AL: "Chalcones: A new class of antimitotic agents" JOURNAL OF MEDICINAL CHEMISTRY, vol. 33, no. 7, juillet 1990 (1990-07), pages 1948-1954, XP001015662 cité dans la demande

## Description

La présente invention concerne de nouveaux composés chimiques, particulièrement de nouveaux aryl-cycloalcanes, des compositions les contenant, et leur utilisation comme médicaments.

Plus particulièrement, l'invention concerne de nouveaux aryl-cycloalcanes présentant une activité anticancéreuse, et en particulier une activité inhibitrice de polymérisation de la tubuline.

Les aryl-cycloalcanes dont il est question ici sont des dérivés carbocycliques de chalcones, dont la chaîne prop-2-èn-1-one est remplacée par une chaîne propanone dont les positions 2 et 3 sont substituées par une chaîne alkyle ou alkényle liée simultanément aux positions 2 et 3 de la chaîne propanone.

Michael L. Edwards *et al.*, article paru dans *J. Med. Chem.* 1990, vol. 33, pp 1948-1954, présentent des chalcones utilisables comme agents antimitotiques.
Le composé 81, présenté dans le tableau IV, est une chalcone linéaire réduite. Son activité anticancéreuse modeste par rapport à son analogue insaturé 36, présenté dans le tableau III, a naturellement conduit les auteurs à se détourner des produits réduits.
En outre, bien que la substitution d'un hydrogène en position 2 sur la chaîne prop-2-èn-1-one entraîne une amélioration substantielle de l'activité (composé 63, tableau III), la substitution d'un hydrogène en position 3 sur la même chaîne anéantit l'activité anti-tumorale (composé 90, tableau IV).

Les chalcones insaturées sont donc de bons candidats pour la préparation de compositions anticancéreuses. Malheureusement, il a été observé que les chalcones insaturées présentaient des déficiences importantes en termes de stabilité. Ainsi, une exposition prolongée à la lumière et/ou à l'air entraîne une dégradation rapide des produits, qui est difficilement compatible avec une utilisation de médicaments après un stockage prolongé d'une durée pouvant aller fréquemment de quelques mois à une ou plusieurs années.

Ainsi, un besoin en dérivés de chalcones présentant simultanément une stabilité accrue et une activité satisfaisante, en particulier vis-à-vis de l'inhibition de la polymérisation de la tubuline, a été ressenti.

Ces exigences sont remplies par les produits de la présente invention, qui répondent à la formule (I) suivante : 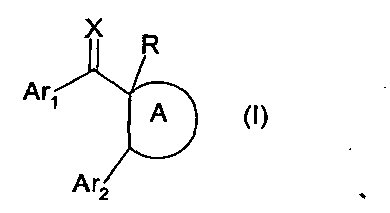 dans laquelle :
- A représente un hydrocarbure cyclique saturé ou partiellement insaturé, contenant de 3 à 14 atomes de carbone, éventuellement substitué;
- X est sélectionné dans le groupe constitué par O,
- R est sélectionné dans le groupe constitué par H, halogène, CH₃, CF₃, C₂H₅, C₃-C₄ alkyle,
- Ar₁ représente un premier noyau phényle, substitué par un substituant ou un groupe de substituants choisis parmi :
   (i) 1 à 4 radicaux OCH₃,
   (ii) un radical méthylènedioxy,
   (iii) un radical éthylènedioxy,
   (iv) 1 ou 2 radicaux OCH₃ et un radical méthylènedioxy,
   (v) 1 ou 2 radicaux OCH₃ et un radical éthylènedixoy.
- Ar₂ est sélectionné dans le groupe constitué par 3-hydroxy-4-méthoxy-phényl ; 4-hydroxy-3-méthoxy-phényl ; 3-hydroxy-4-amino-phényl ; 4-hydroxy-3-amino-phényl; 3-hydroxy-4-(N,N-diméthylamino)-phényl; 4-hydroxy-3-(N,N-diméthylamino)-phényl.

Un des mérites de l'invention est d'avoir découvert qu'il est possible d'obtenir des dérivés de chalcones saturées (ayant un motif 1,3-diaryl-propanone) présentant une activité satisfaisante à l'encontre de la polymérisation de la tubuline.

Un autre des mérites de l'invention est d'avoir découvert qu'il est possible, de façon surprenante, de substituer la position 3 de la chaîne prop-2-èn-1-one par un radical hydrocarboné tout en maintenant une activité satisfaisante à l'encontre de la polymérisation de la tubuline, lorsque ledit radical hydrocarboné est lié à la position 2 de la prop-2-èn-1-one, et que cette dernière est réduite en propanone.

Un autre des mérites de l'invention est d'avoir découvert que, contre toute attente, l'ajout d'un substituant lié simultanément aux positions 2 et 3 d'une 1,3-diaryl-propanone réputée inactive ou très peu active à l'encontre de la polymérisation de la tubuline, conduit à l'obtention de composés inhibant la polymérisation de la tubuline.

De manière préférée, A sera choisi parmi les hydrocarbures monocycliques ou bicycliques, contenant de 3 à 8 atomes de carbone.

A peut être plus préférentiellement un cyclopropyle.

Ar₁ peut être préférentiellement substitué par un groupe de substituants choisis parmi :
(i) 2,5-diméthoxy,
(ii) 3,5-diméthoxy,
(iii) 2,3,4-triméthoxy,
(iv) 3,4,5-triméthoxy,
(v) 2,4,5-triméthoxy,
(vi) 2,3,5-triméthoxy,
(vii) 2,3,4,5-tétraméthoxy,
(viii) 3,4-méthylènedioxy,
(ix) 3,4-éthylènedioxy

Ar₁ sera plus préférentiellement choisi parmi 2,5-diméthoxyphényl et 3,4,5-triméthoxyphényl, ce dernier étant particulièrement préféré.

Un produit conforme à l'invention pourra se présenter sous forme :
1) racémique, ou
2) enrichie en un stéréoisomère, ou
3) enrichie en un énantiomère ;
et pourra être éventuellement salifié.

Un produit conforme à l'invention aura avantageusement la formule (I) suivante : 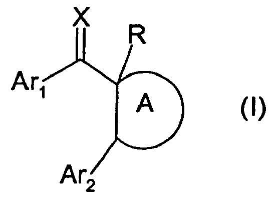 dans laquelle:
- A représente un radical monocycloalkyle ou bicycloalkyle, saturé ou partiellement insaturé, contenant de 3 à 14 atomes de carbone, éventuellement substitué par un ou plusieurs groupes choisis parmi les atomes d'halogènes, ou les radicaux (C1-C7)alkyle, amino, (C1-C7)alkylamino, (C1-C7)dialkylamino, hydroxy, (C1-C7)alcoxy , (C1-C7)alkylthio, carbonyle, cyano, trifluorométhyle, carboxy, (C1-C7)alkoxycarbonyl, carboxamido, N-[(C1-C7)alkyl]carboxamido ou N,N-[(C1-C7)dialkyl]carboxamido, éventuellement substitué par un groupement (C6-C10)aryle condensé, par exemple phényle, naphtyle, ou par un groupement (C2-C13)hétéroaryle condensé, par exemple 1,2,3-triazolo[c]-, pyrido-, carbazolo-, acridino-.

Un produit conforme à l'invention sera utilisé de préférence comme agent inhibant la polymérisation de la tubuline.

Un produit conforme à l'invention sera utilisé de préférence pour favoriser la désagrégation d'amas de cellules provenant d'un tissu vasculaire.

Un produit conforme à l'invention pourra être utilisé pour la fabrication d'un médicament utile pour traiter un état pathologique, en particulier un cancer.

La présente invention concerne aussi les compositions thérapeutiques contenant un composé selon l'invention, en association avec un excipient pharmaceutiquement acceptable selon le mode d'administration choisi. La composition pharmaceutique peut se présenter sous forme solide, liquide ou de liposomes.

Parmi les compositions solides on peut citer les poudres, les gélules, les comprimés. Parmi les formes orales on peut aussi inclure les formes solides protégées vis-à-vis du milieu acide de l'estomac. Les supports utilisés pour les formes solides sont constitués notamment de supports minéraux comme les phosphates, les carbonates ou de supports organiques comme le lactose, les celluloses, l'amidon ou les polymères. Les formes liquides sont constituées de solutions de suspensions ou de dispersions. Elles contiennent comme support dispersif soit l'eau, soit un solvant organique (éthanol, NMP ou autres) ou de mélanges d'agents tensioactifs et de solvants ou d'agents complexants et de solvants.

Les formes liquides seront, de préférence, injectables et, de ce fait, auront une formulation acceptable pour une telle utilisation.

Des voies d'administration par injection acceptables incluent les voies intraveineuse, intra-péritonéale, intramusculaire, et sous cutanée, la voie intraveineuse étant préférée.

La dose administrée des composés de l'invention sera adaptée par le praticien en fonction de la voie d'administration du patient et de l'état de ce dernier.

Les composés de la présente invention peuvent être administrés seuls ou en mélange avec d'autres anticancéreux. Parmi les associations possibles on peut citer :
- les agents alkylants et notamment le cyclophosphamide, le melphalan, l'ifosfamide, le chlorambucil, le busulfan, le thiotepa, la prednimustine, la carmustine, la lomustine, la semustine, la steptozotocine, la decarbazine, la témozolomide, la procarbazine et l'hexaméthylmélamine
- les dérivés du platine comme notamment le cisplatine, le carboplatine ou l'oxaliplatine
- les agents antibiotiques comme notamment la bléomycine, la mitomycine, la dactinomycine
- les agents antimicrotubules comme notamment la vinblastine, la vincristine, la vindésine, la vinorelbine, les taxoides (paclitaxel et docétaxel)
- les anthracyclines comme notamment la doxorubicine, la daunorubicine, l'idarubicine, l'épirubicine, la mitoxantrone, la losoxantrone
- les topoisomérases des groupes I et II telles que l'étoposide, le teniposide, l'amsacrine, l'irinotecan, le topotecan et le tomudex
- les fluoropyrimidines telles que le 5-fluorouracile, l'UFT, la floxuridine
- les analogues de cytidine telles que la 5-azacytidine, la cytarabine, la gemcitabine, la 6-mercaptomurine, la 6-thioguanine
- les analogues d'adénosine telles que la pentostatine, la cytarabine ou le phosphate de fludarabine
- le méthotrexate et l'acide folinique
- les enzymes et composés divers tels que la L-asparaginase, l'hydroxyurée, l'acide trans-rétinoique, la suramine, la dexrazoxane, l'amifostine, l'herceptin ainsi que les hormones oestrogéniques, androgéniques
- les agents antivasculaires tels que les dérivés de la combretastatine ou de la colchicine et leurs prodrogues.

Il est également possible d'associer aux composés de la présente invention un traitement par des radiations. Ces traitements peuvent être administrés simultanément, séparément, séquentiellement. Le traitement sera adapté par le praticien en fonction du malade à traiter.

Un produit conforme à l'invention pourra favoriser la désagrégation d'amas de cellules provenant d'un tissu vasculaire. Plus particulièrement, les produits de la présente invention seront utilisés dans leur première application thérapeutique pour inhiber la croissance des cellules cancéreuses et en même temps la destruction des vaisseaux existants. L'inhibition de la vascularisation est déterminée par un test de détachement cellulaire tel que décrit ci-après.

### Test permettant de déterminer l'inhibition de la vascularisation

Un test de détermination du détachement des cellules endothéliales a été établi afin de sélectionner les produits sur la base de leur activité «in vitro» Ce test de détermination du détachement des cellules endothéliales est caractérisé en ce que les cellules endothéliales, ensemencées dans des plaques dont le fond est recouvert d'un agent liant choisi de préférence parmi la gélatine, la fibronectine ou la vitronectine, après culture, sont additionnées par un milieu contenant le composé à tester, puis les cellules sont marquées avec une substance fluorescente, les cellules qui se sont détachées sont éliminées par lavage et la fluorescence des cellules restantes est comptée au fluorimètre.

Ce test consiste à mesurer le détachement des cellules endothéliales cultivées sur des substrats à base d'un agent liant choisi de préférence parmi la fibronectine, la vitronectine ou la gélatine. De préférence un jour après l'ensemencement des cellules en plaques contenant par exemple 96 puits, le milieu de culture est remplacé par un milieu contenant le composé à tester en absence de sérum. On prépare six fois la même préparation à trois concentrations différentes (0.1, 0.3 et 0.6 µM) et six fois le contrôle sans adjonction de produit antivasculaire. Après deux heures de traitement avec la substance à tester, les cellules sont marquées avec la calcéine-AM (1.6 µg/ml) dans du milieu de culture supplémenté avec 0.1 % de BSA. Les cellules qui se sont détachées sont éliminées par lavage avec le milieu de culture contenant 0.1 % de sérum albumine bovine ; on ajoute 100 µl de milieu à chaque puits. La fluorescence des cellules restantes est comptée au fluorimètre. Les données obtenues sont exprimées par rapport au témoin (cellules non traitées).

L'évaluation du détachement des cellules endothéliales *in vitro* est déterminée de la façon suivante. Les cellules HDMEC (Human Dermal Microvascular Endothelial Cells, Promocell, c-122102) sont cultivées dans un milieu ECGM-MV qui contient 5 % de sérum de veau foetal, des facteurs de croissance (EGF 10 ng/ml, hydrocortisone 1 µg/ml, 0.4 % supplément de croissance avec héparine) et des antibiotiques (amphotericine 50 ng/ml, gentamicine 50 µg/ml). Pour le test de détachement, les HDMEC sont ensemencées à 5,000 cellules dans des plaques 96 puits à fond clair (Costar) pré-coatées avec de la fibronectine (10 µg/ml) ou de la vitronectine (1 µg/ml) ou de la gélatine. Vingt quatre heures plus tard, le milieu de culture est remplacé par du milieu ECGM-MV 0.1 % BSA contenant les produits indiqués. Les concentrations testées sont 0,1-0,3 et 1 µM pour chaque produit. Après deux heures de traitement, les cellules sont marquées pendant une heure à la calcéine (1.6 µg/ml, Molecular Probes) dans du milieu ECGM-MV 0.1 % BSA. Les cellules détachées sont ensuite enlevées par lavage avec du milieu ECGM-MV 0.1 % BSA; 100 µl de milieu est ajouté à chaque puits. La fluorescence des cellules qui restent attachées au substratum du puits est comptée à l'aide d'un fluorimètre, Spectrafluor Plus (Tecan, excitation 485 nm, et émission 535 nm). Les données sont la moyenne de six échantillons différents et sont exprimées en pourcentage du contrôle (cellules non traitées).

Un effet de détachement cellulaire supérieur ou égal à 15 % est considéré comme significatif.

Un produit conforme à l'invention pourra être utile pour inhiber la polymérisation de la tubuline in vitro.

### Evaluation de l'inhibition de polymérisation de tubuline

La tubuline est purifiée à partir de cerveaux de porc selon des méthodes publiées (Shelanski et al., 1973, Proc. Natl. Acad. Sci.USA, **70**, 765-768. Weingarten et al., 1975, Proc. Natl. Acad. Sci.USA, **72**, 1858-1862).

Brièvement, les cerveaux sont broyés et centrifugés dans un tampon d'extraction. La tubuline, contenue dans le surnageant de l'extrait subit deux cycles successifs de polymérisation à 37°C et dépolymérisation à 4°C, avant d'être séparée des MAPs (Microtubule Associated Proteins) par chromatographie sur colonne de phosphocellulose P11 (Whatman). La tubuline, ainsi isolée est pure à plus de 95 %. Elle est conservée dans un tampon nommé RB/2 30 % glycérol, dont la composition est MES-NaOH [acide 2-(N-morpholino)-éthanesulfonique] 50 mM, pH6.8 ; MgCl₂ 0.25 mM ; EGTA 0.5 mM ; glycérol 30 % (v/v), GTP (guanosine-5'-tri-phosphate) 0.2 mM.

La polymérisation de la tubuline en microtubules est suivie par turbidimétrie comme suit : la tubuline est ajustée à une concentration de 10 µM (1 mg/ml) dans le tampon RB/2 30 % glycérol auquel on ajoute 1mM GTP et 6mM MgCl₂. La polymérisation est déclenchée par une augmentation de la température de 6°C à 37°C dans une cuve de 1 cm de trajet optique, placée dans un spectrophotomètre UVIKON 931 (Kontron) équipé d'un porte cuve thermostaté. L'augmentation de la turbidité de la solution est suivie à 350 nm.

Les produits sont mis en solution à 10 mM dans le DMSO et ajoutés à des concentrations variables (0.5 à 10 µM) à la solution de tubuline avant polymérisation. La Cl₅₀ est définie comme la concentration de produit qui inhibe de 50 % la vitesse de polymérisation. On considère comme très actif un produit dont la Cl₅₀ est inférieure ou égale à 3 µM.

### Evaluation de l'inhibition de prolifération de cellules HeLa

On évalue la prolifération des cellules HeLa en mesurant l'incorporation de [¹⁴C]-thymidine de la façon suivante. Les cellules HeLa (cellules épithéliales tumorale d'origine humaine) sont cultivées dans un milieu DMEM (Gibco) qui contient 10 % de sérum de veau foetal et des antibiotiques (pénicilline 1 %, streptomycine 1 %). Pour effectuer le test de prolifération, on ensemence les cellules dans des microplaques cytostar 96 puits (Amersham), à raison de 5000 cellules par puits. On ajoute ensuite la [¹⁴C]-thymidine (0.1 µCi/puits) et les produits à évaluer. On utilise des concentrations variables de produits jusqu'à 10 µM ; le DMSO (solvant utilisé pour solubiliser les produits) ne doit pas excéder 0.5 % dans le milieu. 48 heures après incubation à 37°C, on mesure la radioactivité incorporée dans les cellules par comptage de la plaque dans un compteur TRI-LUX (Wallac). La Cl₅₀ est définie comme la concentration de produit qui diminue de 50 % la radioactivité par rapport à un contrôle non traité. On considère qu'un produit dont la CI₅₀ est inférieure à 1 µM est cytotoxique

### Evaluation de l'effet de détachement de cellules endothéliales HDMEC

L'évaluation du détachement des cellules endothéliales *in vitro* est déterminée de la façon suivante : Des cellules HDMEC (Human Dermal Microvascular Endothelial Cells, Promocell, c-122102) sont cultivées dans un milieu ECGM-MV qui contient 5 % de sérum de veau foetal, des facteurs de croissance (EGF 10 ng/ml, hydrocortisone 1 µg/ml, 0.4 % supplément de croissance avec héparine) et des antibiotiques (amphotéricine 50 ng/ml, gentamicine 50 µg/ml) Pour le test de détachement, les HDMEC sont ensemencées à 5,000 cellules dans des plaques 96 puits à fond clair (Costar) pré-coatées avec de la fibronectine (10 µg/ml) ou de la vitronectine (1 µg/ml) ou de la gélatine. Vingt quatre heures plus tard, le milieu de culture est remplacé par du milieu ECGM-MV 0.1 % BSA contenant les produits indiqués. Les concentrations testées sont 0,1-0,3 et 1 µM pour chaque produit. Après deux heures de traitement, les cellules sont marquées pendant une heure à la calcéine (1.6 µg/ml, Molecular Probes) dans du milieu ECGM-MV 0.1 % BSA. Les cellules détachées sont ensuite enlevées par lavage avec du milieu ECGM-MV 0.1 % BSA; 100 µl de milieu est ajouté à chaque puits. La fluorescence des cellules qui restent attachées au substratum du puits sont comptées à l'aide d'un fluorimètre, Spectrafluor Plus (Tecan, excitation 485 nm, et émission 535 nm). Les données sont la moyenne de six échantillons différents et sont exprimées en pourcentage du contrôle (cellules non traitées).
Un effet de détachement cellulaire supérieur ou égal à 15 % est considéré comme significatif.

### Définitions

« Halogène » est un élément choisi parmi F, Cl, Br, et l.
« Cycloalkyl » peut être un substituant choisi parmi cyclopropyl, cyclobutyl, cyclopentyl, cyclohexyl, cycloheptyl, bicyclo[2.2.1]heptyl, bicyclo[2.2.2]octyl.
« Cycloalkylalkyl » est un substituant alkyl, lui-même substitué par un groupement cycloalkyl, par exemple tel que défini ci-dessus.
   Un substituant défini par « Hétérocycle azoté ayant de 5 à 12 atomes de carbone» peut être choisi dans le groupe constitué par : pyridin-3-yle, pyridin-4-yle, pyrimidin-4-yle, quinoléin-6-yle, quinoléin-7-yle, isoquinoléin-6-yle, isoquinoléin-7-yle, quinazolin-6-yle, quinoxalin-6-yle, N-alkyl-indol-5-yle, N-alkyl-indol-6-yle, N-alkyl-tétrahydro-1,2,3,4-quinoléin-6-yle, N-alkyl-tétrahydro-1,2,3,4-quinoléin-7-yle, acridin-2-yle, acridin-3-yle, N-alkyl-carbazol-2-yle ou N-alkylcarbazol-3-yle.
« Aryl » est un substituant comprenant au moins un cycle aromatique, ce dernier comprenant éventuellement un ou plusieurs hétéroatomes participant à l'aromaticité. Un exemple de groupe aryl peut être un substituant choisi parmi phényl, naphtyl, indenyl, pyridyl, pyrimidinyl, pyrazinyl, thiényl, furyl, pyrolyl, thiazolyl, oxazolyl, thiadiazolyl, oxadiazolyl, imidazolyl, quinoléyl, isoquinoléyl, benzothiényl, benzofuryl, indolyl, benzothiazolyl, benzoxazolyl, benzimidazolyl, indazolyl, azaindolyl.
« Hétéroaryl » est un groupement aryl comprenant au moins un hétéroatome participant à l'aromaticité. De ce fait, les groupements aryles tels que phényl, naphtyl, anthracényl, pyrènyl, qui sont dépourvus d'hétéroatomes intracycliques, ne font pas partie du groupe des hétéroaryl.
« Aralkyl » est un substituant alkyl, lui-même substitué par un groupement aryl. Un groupement benzyl est un exemple de substituant aralkyl.
   Le terme «substitué» notamment présent dans les expressions « alkyl substitué », et « aralkyl substitué », se rapporte nécessairement à un substituant différent de H, qui peut être choisi parmi alkyl, F, Cl, Br, I, N(R7, R8), N(O)(R7, R8), NO, NO₂, O(R7), S(R7), SO(R7), SO₂(R7), OSO₂(R7), PO₃(R7), OPO₃(R7), CO(R7), COO-(R7), CONH-(R7), CON(R7, R8), CN, C≡C-(R7), N=C-(R9), aryl, aralkyl ; dans lequel R7, R8 sont indépendamment sélectionnés dans le groupe constitué par H, alkyl, alkylène, aryl, aralkyl, C(O)-(R7), C(S)-(R7), alkyl-O(R7), alkyl-S(R7), alkyl-N(R7, R8), dans lequel lorsque R7, R8 sont simultanément présents, ils peuvent être liés entre eux pour former un cycle, R9 est sélectionné dans le groupe constitué par alkyl, alkylène, aryl, aralkyl, alkyl-O(R7), alkyl-S(R7), alkyl-N(R7, R8); et à tous les autres substituants acceptables connus de l'homme de l'art à la date de dépôt de l'invention.
« Acide aminé » comprend les acides aminés naturels et artificiels, sous forme énantiomériquement pure ou en mélange.
« Aminoacide » ou « amino-acide » sont des synonymes de « acide aminé ».
« Hétérocycle azoté de 5 à 12 chaînons » peut être un hétérocycle sélectionné dans le groupe constitué par: pyridin-3-yle, pyridin-4-yle, pyrimidin-4-yle, quinolin-6-yle, quinolin-7-yle, isoquinolin-6-yle, isoquinol-7-yle, quinazolin-6-yle, quinoxalin-6-yle, N-alkyl-indol-5-yle, N-alkyl-indol-6-yle, N-alkyl-tétrahydro-1,2,3,4-quinolin-6-yle, N-alkyl-tétrahydro-1,2,3,4-quinolin-7-yle, acridin-2-yle, acridin-3-yle, N-alkyl-carbazol-2-yle, N-alkylcarbazol-3-yle

Les produits de formule générale (I), 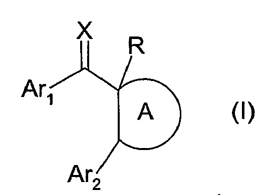 définis précédemment peuvent être obtenus par réaction de cycloaddition, de type 2+1 ou 2+2 ou 2+3 ou 2+4, à partir d'une chalcone de formule générale (II), dans laquelle R, Ar₁ et Ar₂ sont définis tels que ci-dessus et X représente un atome d'oxygène selon le schéma général (I) ci-dessous, pour conduire aux produits de formule générale (I) dans lesquels X représente un atome d'oxygène.

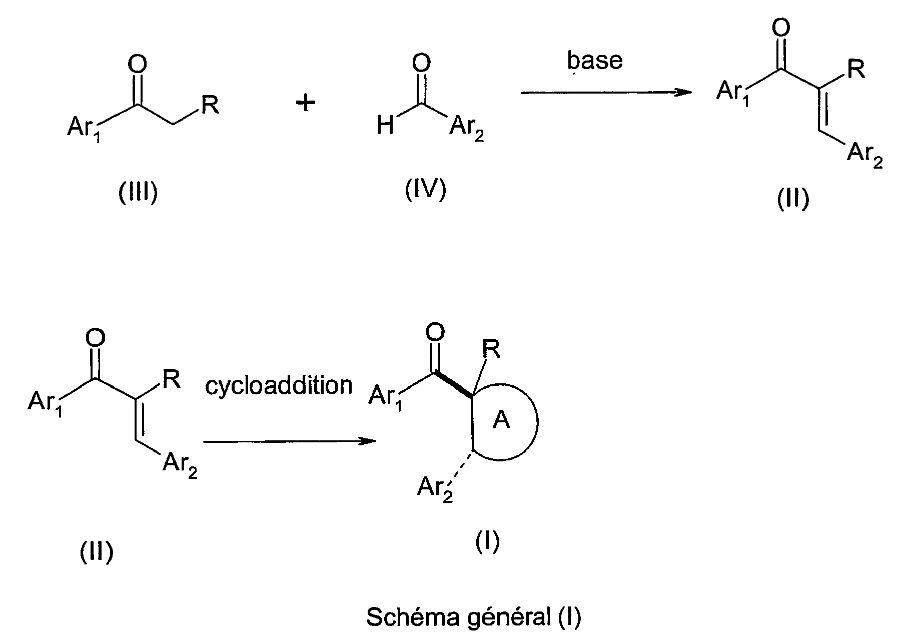

Il est possible d'utiliser les méthodes de cycloaddition, de type 2+1 ou 2+2 ou 2+3 ou 2+4 connues de l'homme de l'art et plus particulièrement celles décrites dans :
- Advances in cycloaddition Vol I (1988) (éditeur D. Curran),
- Advances in cycloaddition Vol II (1990) (éditeur D. Curran),
- Advances in cycloaddition Vol III (1993) (éditeur D. Curran),
- Advances in cycloaddition Vol IV (1997) (éditeur M. Lauters),
- Advances in cycloaddition Vol I (1999) (éditeur M. Harmaka),
- 1,3 Dipolar cycloaddition Vol I & Vol II (1984) (éditeur A. Padwa).

Lorsque Ar₂ représente un noyau phényle substitué par un radical hydroxy ou amino, il est particulièrement avantageux de protéger cette fonction préalablement à la réaction de cycloaddition, puis de la déprotéger dans les conditions décrites dans Protective Groups in Organic Chemistry 1981 (T. Greene, Wiley éditeur). Dans le cas où ledit substituant est un radical hydroxy, on utilisera de préférence un groupement silylé tel que le groupement tert.butyldiméthylsilyl (TBDMS), lorsque ledit substituant représente un radical aminé on utilisera de préférence un radical nitro ou un groupe alkyloxycarbonyl tel que tert-butyloxycarbonyl (Boc) ou benzyloxycarbonyl (Cbz).

Dans le cas où A représente un radical cyclopropyle, il est particulièrement avantageux de faire réagir la chalcone de formule générale (II) avec un réactif comme, à titre non limitatif, un ylure de sulfoxonium ou de sulfonium ou un ylure de phosphonium ou un dérivé de diazométhane ou un dérivé malonique ou un carbène généré in situ.

Dans le cas où A représente un radical cyclobutyle, il est particulièrement avantageux de faire réagir la chalcone de formule générale (II) avec un dérivé éthylènique riche en électrons comme, à titre non limitatif, un 1,1-dialkyle-éthylène ou 1,1-dialcoxy-éthylène ou un trialkylsilyloxy-éthylène ou un 3-trialkylstannyl-propène.

Dans le cas où A représente un radical cyclopentyle, il est particulièrement avantageux de faire réagir la chalcone de formule générale (II) avec un dérivé « équivalent de triméthylèneméthane », en présence de catalyseur au palladium, comme, à titre non limitatif, celui décrit dans J. Amer. Chem. Soc. (1983), 105, 2315.

Dans le cas où A représente un radical cyclopentyle, il est particulièrement avantageux de faire réagir la chalcone de formule générale (II) avec un dérivé de butadiène.

Dans le cas où A représente un radical bicyclo[2.2.1]heptyle ou de bicyclo[2.2.2]octyle, il est particulièrement avantageux de faire réagir la chalcone de formule générale (II) avec respectivement un dérivé de cyclopentadiène ou de 1,3-cyclohexadiène.

Il est entendu que les réactions de cycloaddition sur une chalcone peuvent être réalisées de manière énantiosélective ou énantiospécifique en utilisant soit un réactif soit un catalyseur porteur de chiralité.

Il est également entendu que, dans la mesure où la cycloaddition conduit à l'obtention d'un produit présentant une insaturation, cette dernière peut être réduite par une des méthodes conventionnelles largement décrites, par exemple une hydrogénation catalytique utilisant un métal de transition, notamment le palladium, éventuellement en présence d'un poison modérateur d'activité permettant une hydrogénation sélective, sur un support acceptable, par exemple du charbon activé, de l'alumine ou de l'amiante.

Les chalcones de formule générale (I) sont généralement préparées par couplage, en présence d'une base et d'un agent de déshydratation, entre une cétone aromatique de formule générale (III) et un aldéhyde aromatique de formule générale (IV), selon, par exemple et à titre non limitatif, J. Med. Chem. , 1990, 33, 1948.
Les cétones aromatiques de formule générale (III) et les aldéhydes de formule générale (IV) sont soit commerciaux, soit décrits dans la littérature, soit préparés par des méthodes classiques de synthèse des cétones ou aldéhydes aromatiques connues de l'homme de l'art.

Selon un second schéma général de synthèse, les produits de formule générale (I) peuvent être préparés à partir de dérivés de 2-aryl-cycloalkanecarboxylates de formule générale (V) dans lesquels A, R et Ar₂ sont définis tels que précédemment. Deux voies différentes sont alors possibles :
- soit l'acylation, de type Friedel-Crafts, d'un produit de formule générale Ar₁H par un dérivé de formule générale (V) dans lequel X représente un atome d'halogène, préférentiellement un atome de chlore, ou un radical hydroxy, en présence d'un catalyseur acide soit minéral, comme de l'acide polyphosphorique ou de l'acide trifluoroacétique ou de l'acide trifluorométhanesulfonique, soit un acide de Lewis comme le trichlorure d'aluminium, le tétrachlorure d'étain ou de titane ou le trifluorure de bore en complexe avec de l'oxyde de diéthyle. Cette méthode est particulièrement intéressante dans la mesure ou la partie Ar₁ des produits de formule générale (I) correspond à des noyaux aromatiques riches en électrons et donc très réactifs dans des réactions d'acylations de type Friedel-Crafts ;
- soit la réaction d'un organométallique, de préférence un organomagnésien ou un organolithien, avec un dérivé de formule générale (V), dans lequel X représentera un atome d'halogène, de préférence un atome de chlore, ou un radical hydroxy, ou un radical NR₁R₂, dans lequel R₁ et R₂ sont préférentiellement différent d'un atome d'hydrogène et dans lequel, très préférentiellement, R₁ représente un radical méthyl et R₂ représente un radical méthoxy pour former dérivé connu sous le nom d' « amide de Weinreb ».
Ar₁H / Friedel-Crafts
X = Cl, OH

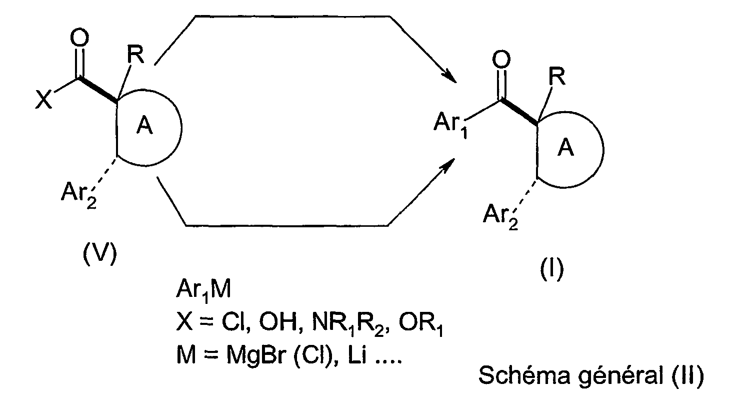

Les produits de formule générale (V) sont soit connus, soit préparés selon les méthodes connues de synthèse des 2-aryl-cycloalkanecarboxylates.

A titre d'exemples non limitatifs :
- les 2-aryl-cyclopropanecarboxylates peuvent être préparés par cycloaddition de type 2+1 d'un dérivé de cinnamate, en utilisant les méthodes décrites dans le schéma général (I), ou par couplage électrochimique d'un dérivé de cinnamate avec du dibromométhane, selon J. Org. Chem., 1990, 55, 2503, ou par réaction d'un dérivé de stilbène avec le diazoacétate d'éthyle selon J. Chem. Soc., Chem. Commun., 1985, 328 ;
- les 2-aryl-cyclobutanecarboxylates peuvent être préparés par cycloaddition de type 2+2 d'un dérivé de cinnamate, en utilisant les méthodes décrites dans le schéma général (I), ou par couplage électrochimique d'un dérivé de cinnamate avec un 1,2-dibromoéthane, selon J. Org. Chem., 1990, 55, 2503,
ou par décarboxylation d'un 2-aryl-cyclobutane-1,1-dicarboxylate selon J. Het. Chem., 1995, 1493 ;
- les 2-aryl-cyclopropanecarboxylates peuvent être préparés par cycloaddition de type 2+1 d'un dérivé de cinnamate, en utilisant les méthodes décrites dans le schéma général (I), ou par couplage électrochimique d'un dérivé de cinnamate avec un 1,3-dibromopropane, selon J. Org. Chem., 1990, 55, 2503, ou par arylation énantiosélective d'un cyclopent-1-ène-carboxylate avec un aryllithien, selon J. Org. Chem., 1992, 57, 4300 ;
- les 2-aryl-cyclohexanecarboxylates peuvent être préparés par cycloaddition de type 2+1 d'un dérivé de cinnamate, en utilisant les méthodes décrites dans le schéma général (I), ou par couplage électrochimique d'un dérivé de cinnamate avec un 1,4-dibromobutane, selon J. Org. Chem., 1990, 55, 2503, ou par arylation énantiosélective d'un cyclohex-1-ène-carboxylate avec un aryllithien, selon J. Org. Chem., 1992, 57, 4300, ou par cycloaddition de type Diels-Alder entre un 4-aryl-butadiène et un acrylate, selon le brevet US 5.866.513.

Les composés aromatiques de formule générale Ar₁H sont soit commerciaux soit préparés selon les méthodes décrites dans la littérature, les composés organométalliques de formule générale Ar₁M sont soit commerciaux soit préparés à partir des dérivés halogénés, de préférence bromés ou chlorés, correspondants, eux-mêmes commerciaux ou préparés selon les méthodes décrites dans la littérature.

### Exemple 1 : [2-(3-Hydroxy-4-méthoxy-phényl)-cyclopropyl]-(3,4,5-triméthoxy-phényl)-méthanone, racémate trans

Etape 1 : Dans un tricol de 250 mL, 5 g de E-3-(3-hydroxy-4-méthoxy-phényl)-1-(3,4,5-triméthoxy-phényl)-propènone, qui peuvent être obtenus selon Bioorg. Med. Chem. Lett. (1998), 8, 1051, sont dissous dans 75 mL de DMF. On ajoute alors 2,4 g de tert.butyldiméthylchlorosilane et 1,08 g d'imidazole. Après 20 heures d'agitation à température ambiante, le milieu réactionnel est coulé sur 400 mL d'eau. On extrait 3 fois par 75 mL d'acétate d'éthyle, puis les phases organiques jointes sont lavées à l'eau, séchées sur sulfate de magnésium et concentrées sous pression réduite. On obtient alors 6,6 g de E-3-(3-tert.butyldiméthylsilyloxy-4-méthoxy-phényl)-1-(3,4,5-triméthoxy-phényl)-propènone, utilisée telle quelle à l'étape suivante.

Etape 2 : Dans un tricol de 50 mL, on ajoute, sous atmosphère d'argon, 124 mg d'hydrure de sodium, en suspension à 60 % dans l'huile, et 10 mL de DMSO. Puis on ajoute par portions 660 mg d'iodure de triméthylsulfoxonium et on agite pendant 1 heure après cessation du dégagement gazeux. On ajoute ensuite, par portions en 15 minutes, 1,38 g de E-3-(3-tert.butyldiméthylsilyloxy-4-méthoxy-phényl)-1-(3,4,5-triméthoxy-phényl)-propènone, et on agite pendant 20 heures à température ambiante. Le milieu réactionnel est alors jeté sur 200 mL d'eau et 100 g de glace pilée, puis extrait 3 fois par 75 mL d'acétate d'éthyle. Les phases organiques jointes sont lavées à l'eau et séchées sur sulfate de magnésium. Après concentration sous pression réduite, le résidu huileux orange obtenu est purifié par flash-chromatographie sur gel de silice (70-230 Mesh) en éluant par un mélange de cyclohexane et d'acétate d'éthyle (70-30 en volumes). On obtient ainsi 0,9 g d'une gomme jaune qui est recristallisée dans 5 mL d'oxyde de diisopropyle, pour donner 650 mg de [2-(3-Hydroxy-4-méthoxy-phényl)-cyclopropyl]-(3,4,5-triméthoxy-phényl)-méthanone, racémate trans pur, dont les caractéristiques sont les suivantes :
- point de fusion (Kofler) = 75°C
- analyse élémentaire : %C = 66,43% ; %H = 6,15%.

### Exemple 2 : [2-(4-diméthylamino-phényl)-cyclopropyl]-(3,4,5-triméthoxy-phényl)-méthanone, racémate trans

En opérant comme à l'étape 2 de l'exemple 1, mais à partir de 1 g de de E-3-(4-diméthylamino-phényl)-1-(3,4,5-triméthoxy-phényl)-propènone, qui peut être obtenue selon J. Med. Chem. (1990), 33, 1948, on obtient, après purification par flash-chromatographie sur gel de silice (70-230 Mesh) en éluant par un mélange de cyclohexane et d'acétate d'éthyle (70-30 en volumes), 650 mg de [2-(4-diméthylamino-phenyl)-cyclopropyl]-(3,4,5-trimethoxy-phenyl)-methanone, racémate trans pur, sous forme d'une huile dont la caractéristique est la suivante :
Spectre de RMN . ¹H (300 MHz, (CD₃)₂SO, δ en ppm) : 1,51 (mt : 1H); 1,64 (mt : 1H) ; 2,47 (mt : 1H); 2,88 (s : 6H) ; 3,11 (mt : 1H) ; 3,76 (s : 3H) ; 3,86 (s : 6H) ; 6,69 (d large, J = 9 Hz : 2H) ; 7,11 (d large, J = 9 Hz : 2H) ; 7,34 (s : 2H).

### Exemple 3 : [2-(3-Hydroxy-4-méthoxy-phényl)-bicyclo[2.2.1]hept-4-ènyl]-(3,4,5-triméthoxy-phényl)-méthanone, racémate trans

Etape 1 : Dans un tricol de 25 mL, on ajoute, sous atmosphère d'argon, 566 mg de cyclopentadiène, fraîchement distillé et 1 mL de dichlorométhane, puis on refroidit à 0° et on ajoute 1,07 g de tétrachlorure d'étain, sous forme de pentahydrate. Puis on coule, goutte à goutte à 0°C, 1,38 g de E-3-(3-tert.butyldiméthylsilyloxy-4-méthoxy-phényl)-1-(3,4,5-triméthoxy-phényl)-propènone, préparée à l'étape 1 de l'exemple 1, en solution dans 4 mL de dichlorométhane. Après 4 heures d'agitation à 0°C, on laisse revenir à température ambiante et on agite pendant 16 heures supplémentaires à l'ambiante. Après addition de 10 mL d'eau et de 5 mL de dichlorométhane, on ajuste le pH à 8 par addition d'une solution saturée de bicarbonate de sodium. La phase organique est séparée, puis la phase aqueuse est réextraite 2 fois par 10 mL de dichlorométhane. Les phases organiques jointes sont lavées à l'eau, séchées sur sulfate de sodium et concentrées sous pression réduite. Le produit brut est purifié par flash-chromatographie sur gel de silice (70-230 Mesh) en éluant par un mélange de cyclohexane et d'acétate d'éthyle (80-20 en volumes). On obtient ainsi 700 mg de [2-(3-tert.butyldiméthylsilyloxy-4-méthoxy-phenylrbicyclo[2.2.1 1]hept-4-ènyl]-(3,4,5-triméthoxy-phényl)-méthanone, racémate trans contenant environ 10 % de racémate cis, sous forme d'une huile jaune utilisée telle quelle à l'étape suivante.

Etape 2: Dans un tricol de 25 mL, une solution de 700 mg de [2-(3-tert.butyldiméthylsilyloxy-4-méthoxy-phényl)-bicyclo[2.2.1]hept-4-ènyl]-(3,4,5-triméthoxy-phényl)-méthanone dans 10 mL de THF est refroidie à 0°C. On ajoute alors 1,49 mL d'une solution 1M de fluorure de tétrabutyl ammonium dans le THF. Après une heure d'agitation à 0°C, on laisse revenir à température ambiante et on agite 5 heures supplémentaires à l'ambiante. Le milieu réactionnel est ensuite coulé dans 50 mL d'eau, puis on extrait 3 fois par 20 mL d'acétate d'éthyle. Les phases organiques jointes sont lavées à l'eau, séchées sur sulfate de magnésium et concentrées sous pression réduite. Le produit brut est purifié par flash-chromatographie sur gel de silice (70-230 Mesh) en éluant par un mélange de cyclohexane et d'acétate d'éthyle (70-30 en volumes). On obtient ainsi 270 mg de [2-(3-hydroxy-4-méthoxy-phenyl)-bicyclo[2.2.1]hept-4-ènyl]-(3,4,5-trimethoxy-phenyl)-méthanone, racémate trans contenant environ 5 % de racémate cis, sous forme d'une poudre crème dont les caractéristiques sont les suivantes :
- point de fusion (Kofler) = 152°C
- analyse élémentaire : % C = 70,38 ; % H = 6,09.

**RESULTATS BIOLOGIQUES**

| Exemple N° | Tubuline : Inhibition de polymérisation Cl₅₀ (µM) | Cellules HeLa : Inhibition de prolifération Cl₅₀ (µM) | Cellules HDMEC : Inhibition de proliferation Cl₅₀(µM) | Pourcentage de détachement de cellules HDMEC induit par le composé cité en exemple à une concentration de 1 µM |
|---|---|---|---|---|
| 1 | 1,0 | <0,0019 | 0.017-0.018 | 36-37 |
| 2 | 2,5 | 1,35/1,45 | 4 | nd |
| 3 | 4,5 | 0,52-1,06 | nd | nd |

| | | | | |
|---|---|---|---|---|
| nd: non déterminé | | | | |

## Revendications

1. Produit répondant à la formule (I) suivante : 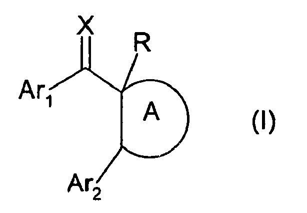 dans laquelle :
- A représente un hydrocarbure cyclique saturé ou partiellement insaturé, contenant de 3 à 14 atomes de carbone, éventuellement substitué;
- X est sélectionné dans le groupe constitué par O,
- R est sélectionné dans le groupe constitué par H, halogène, CH₃, CF₃, C₂H₅, C₃-C₄ alkyle,
- Ar₁ représente un premier noyau phényle, substitué par un substituant ou un groupe de substituants choisis parmi :
(i) 1 à 4 radicaux OCH₃,
(ii) un radical méthylènedioxy,
(iii) un radical éthylènedioxy,
(iv) 1 ou 2 radicaux OCH₃ et un radical méthylènedioxy,
(v) 1 ou 2 radicaux OCH₃ et un radical éthylènedixoy.
- Ar₂ est sélectionné dans le groupe constitué par 3-hydroxy-4-méthoxy-phényl ; 4-hydroxy-3-méthoxy-phényl ; 3-hydroxy-4-amino-phényl ; 4-hydroxy-3-amino-phényl ; 3-hydroxy-4-(N,N-diméthylamino)-phényl ; 4-hydroxy-3-(N,N-diméthylamino)-phényl.

2. Produit selon la revendication 1, **caractérisé en ce que** A est un hydrocarbure monocyclique ou bicyclique, contenant de 3 à 8 atomes de carbone.

3. Produit selon la revendication 2, **caractérisé en ce que** A est un cyclopropyle.

4. Produit selon la revendication 1, **caractérisé en ce que** Ar₁ est substitué par un groupe de substituants choisis parmi :
(i) 2,5-diméthoxy,
(ii) 3,5-diméthoxy,
(iii) 2,3,4-triméthoxy,
(iv) 3,4,5-triméthoxy,
(v) 2,4,5-triméthoxy,
(vi) 2,3,5-triméthoxy,
(vii) 2,3,4,5-tétraméthoxy,
(viii) 3,4-méthylènedioxy,
(ix) 3,4-éthylènedioxy.

5. Produit selon l'une quelconque des revendications précédentes, **caractérisé en ce qu'**il est sous forme:
4) racémique, ou
5) enrichie en un stéréoisomère, ou
6) enrichie en un énantiomère ;
et **en ce qu'**il est éventuellement salifié.

6. Composition pharmaceutique comprenant un produit selon l'une quelconque des revendications précédentes, en combinaison avec un excipient pharmaceutiquement acceptable.

7. Utilisation d'un produit selon l'une quelconque des revendications 1 à 5, comme agent inhibant la polymérisation de la tubuline in-vitro.

8. Utilisation d'un produit selon l'une quelconque des revendications 1 à 5, pour favoriser la désagrégation d'amas de cellules provenant d'un tissu vasculaire in-vitro.

9. Utilisation d'un produit selon l'une quelconque des revendications 1 à 5, pour la fabrication d'un médicament utile pour traiter un état pathologique.

10. Utilisation selon la revendication 9, dans laquelle l'état pathologique est le cancer.

## Claims

1. Product corresponding to formula (I) below: 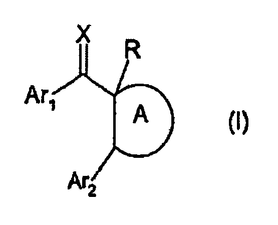 in which:
- A is an optionally substituted, saturated or partially unsaturated, cyclic hydrocarbon containing from 3 to 14 carbon atoms;
- X is selected from the group comprising O,
- R is selected from the group comprising H, halogen, CH₃, CF₃, C₂H₅, and C₃-C₄ alkyl,
- Ar₁ is a first phenyl ring substituted with a substituent or a group of substituents selected from:
(i) 1 to 4 OCH₃ radicals,
(ii) a methylenedioxy radical,
(iii) an ethylenedioxy radical,
(iv) 1 or 2 OCH₃ radical (s) and a methylenedioxy radical,
(v) 1 or 2 OCH₃ radical (s) and an ethylenedioxy radical,
- Ar₂ is selected from the group consisting of 3-hydroxy-4-methoxyphenyl; 4-hydroxy-3-methoxyphenyl; 3-hydroxy-4-aminophenyl; 4-hydroxy-3-aminophenyl; 3-hydroxy-4-(N,N-dimethylamino)-phenyl; and 4-hydroxy-3-(N,N-dimethylamino)-phenyl.

2. Product according to Claim 1, **characterized in that** A is a monocyclic or bicyclic hydrocarbon containing from 3 to 8 carbon atoms.

3. Product according to Claim 2, **characterized in that** A is a cyclopropyl.

4. Product according to Claim 1, **characterized in that** Ar₁ is substituted with a group of substituents selected from:
(i) 2,5-dimethoxy,
(ii) 3,5-dimethoxy,
(iii) 2,3,4-trimethoxy,
(iv) 3,4,5-trimethoxy,
(v) 2,4,5-trimethoxy,
(vi) 2,3,5-trimethoxy,
(vii) 2,3,4,5-tetramethoxy,
(viii) 3,4-methylenedioxy,
(ix) 3,4-ethylenedioxy.

5. Product according to any one of the preceding claims, **characterized in that** it is in:
4) a racemic form, or
5) in a form enriched in a stereoisomer, or
6) in a form enriched in an enantiomer;
and **in that** it is optionally salified.

6. Pharmaceutical composition comprising a product according to any one of the preceding claims, in combination with a pharmaceutically acceptable excipient.

7. Use of a product according to any one of Claims 1 to 5, as an agent for inhibiting tubulin polymerization in vitro.

8. Use of a product according to any one of Claims 1 to 5, for promoting the disaggregation of clumps of cells originating from a vascular tissue in vitro.

9. Use of a product according to any one of Claims 1 to 5, for the manufacture of a medicament of use in the treatment of a pathological condition.

10. Use according to Claim 9, in which the pathological condition is cancer.

## Patentansprüche

1. Produkt der folgenden Formel: 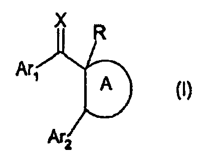 in der
- A einen gegebenenfalls substituierten gesättigten oder ungesättigten zyklischen Kohlenwasserstoff mit 3 bis 14 Kohlenwasserstoffatomen bedeutet;
- X aus der aus O bestehenden Gruppe stammt;
- R aus der aus H, Halogen, CH₃, CF₃, C₂H₅ und C₃-C₄-Alkyl bestehenden Gruppe stammt;
- Ar₁ einen ersten Phenylring bedeutet, der durch einen Substituenten bzw. eine Substituentengruppe aus der Reihe
(i) 1 bis 4 OCH₃-Reste,
(ii) ein Methylendioxyrest,
(iii) ein Ethylendioxyrest,
(iv) 1 oder 2 OCH₃-Reste und ein Methylendioxyrest,
(v) 1 oder 2 OCH₃-Reste und ein Ethylendioxyrest
substituiert ist; und
- Ar₂ aus der Gruppe 3-Hydroxy-4-methoxyphenyl; 4-Hydroxy-3-methoxyphenyl; 3-Hydroxy-4-aminophenyl; 4-Hydroxy-3-aminophenyl; 3-Hydroxy-4-(N,N-dimethylamino)phenyl und 4-Hydroxy-3-(N,N-dimethylamino)phenyl stammt.

2. Produkt nach Anspruch 1, **dadurch gekennzeichnet, daß** A ein monocyclischer oder bicyclischer Kohlenwasserstoff mit 3 bis 8 Kohlenstoffatomen ist.

3. Produkt nach Anspruch 2, **dadurch gekennzeichnet, daß** A Cyclopropyl ist.

4. Produkt nach Anspruch 1, **dadurch gekennzeichnet, daß** Ar₁ durch eine Substituentengruppe aus der Reihe
(i) 2,5-Dimethoxy,
(ii) 3,5-Dimethoxy,
(iii) 2,3,4-Trimethoxy,
(iv) 3,4,5-Trimethoxy,
(v) 2,4,5-Trimethoxy,
(vi) 2,3,5-Trimethoxy,
(vii) 2,3,4,5-Tetramethoxy,
(viii) 3,4-Methylendioxy,
(ix) 3,4-Ethylendioxy
substituiert ist.

5. Produkt nach einem der vorhergehenden Ansprüche, **dadurch gekennzeichnet, daß** es
4) in racemischer Form oder
5) in an einem Stereoisomer angereicherter Form oder
6) in an einem Enantiomer angereicherter Form vorliegt
und daß es gegebenenfalls in Salzform vorliegt.

6. Pharmazeutische Zusammensetzung, die ein Produkt nach einem der vorhergehenden Ansprüche in Kombination mit einem pharmazeutisch unbedenklichen Grundstoff enthält.

7. Verwendung eines Produkts nach einem der Ansprüche 1 bis 5 als in-vitro-Hemmstoff für die Polymerisation von Tubulin.

8. Verwendung eines Produkts nach einem der Ansprüche 1 bis 5 zur Förderung der Freisetzung von Zellen aus einer Zellmasse, die aus einem in vitro kultivierten Gefäßgewebe stammt.

9. Verwendung eines Produkts nach einem der Ansprüche 1 bis 5 zur Herstellung eines Arzneimittels zur Behandlung eines pathologischen Zustands.

10. Verwendung nach Anspruch 9, wobei es sich bei dem pathologischen Zustand um Krebs handelt.
